# EUROPÄISCHE PATENTSCHRIFT

(11) **EP 2 213 254 B1**
(45) Veröffentlichungstag und Bekanntmachung des Hinweises auf die Patenterteilung: **21.03.2012**
(21) Anmeldenummer: 10000526.3
(22) Anmeldetag: 20.01.2010
(51) Int. Cl.: A61B 17/295

(54) **Chirurgisches Instrument**
Surgical instrument
Instrument chirurgical

(30) Priorität: 29.01.2009 DE 102009006689; 23.03.2009 DE 202009003869 U
(43) Veröffentlichungstag der Anmeldung: 04.08.2010
(73) Patentinhaber: Heinemann, Norbert, 78549 Spaichingen (DE)
(72) Erfinder: Heinemann, Norbert, 78549 Spaichingen (DE)
(74) Vertreter: Wagner, Kilian

(56) Entgegenhaltungen:
- DE-A1- 4 316 768
- DE-A1- 19 702 079
- DE-U1- 29 924 518
- US-A1- 2004 044 346

## Beschreibung

Die Erfindung betrifft ein chirurgisches Instrument, insbesondere einen Laminektomie Rongeur oder ein Conchotom, gemäß dem Oberbegriff des Anspruchs 1.

Derartige, auch als Schiebeschaftinstrumente bezeichnete chirurgische Instrumente sind hinlänglich bekannt. In der Regel werden Laminektomie Rongeure als Stanze zum Durchtrennen von Gewebe, Knorpel oder Knochen eingesetzt und werden vornehmlich bei Wirbelsäulenoperationen verwendet. Schiebeschaftinstrumente zeichnen sich durch ein relativ zu einem feststehenden Schaft längsverschiebliches Schiebeteil aus, welches durch Betätigen eines verschwenkbaren Griffteils (Griffbranche) in Richtung der Längserstreckung des Schaftes verschiebbar ist. Üblicherweise wirken dabei das Schiebeteil und der Schaft mit distalen Arbeitsenden in der Art einer Stanze zusammen.

Problematisch bei bekannten chirurgischen Instrumenten ist meist deren nur unzulängliche und aufwändige Reinigbarkeit. Um Schiebeschaftinstrumente verbessert reinigen zu können, insbesondere in einem Bereich zwischen Schiebeteil und Schaft, in welchen bei der Operation biologisches Material eintreten kann, sind Schiebeschaftinstrumente bekannt geworden, bei denen das Schiebeteil vollständig oder teilweise um eine quer zur Längserstreckung des Schaftes sowie parallel zur Schwenkachse des Gritteils verlaufende Drehachse verschwenkbar anzuordnen, um hiermit die Zugänglichkeit einer Schiebeteilführung zu Reinigungszwecken zu optimieren. Schiebeschaftinstrumente mit um eine Drehachse verschwenkbarem Schiebeteil sind beispielsweise in der DE 10 2006 043 970 A1, der DE 299 24 518 U1 oder der US 5,961,531 A beschrieben. Nachteilig bei den bekannten chirurgischen Instrumenten ist, dass das Schiebeteil nicht mittels Einhandbedienung aus einer Arbeitsstellung in eine Reinigungsstellung, in welcher es um die Drehachse verschwenkbar ist, überführbar ist. Ferner ist von Nachteil, dass bekannte chirurgische Instrumente teilweise zu Reinigungszwecken zerlegt werden müssen, also das Schiebeteil Reinigungszwecken zerlegt werden müssen, also das Schiebeteil nach Verschwenken um die Drehachse vom Schaffteil entnommen werden muss.

Neben den zuvor beschriebenen Laminektomie Rongeuren sind als Conchotom ausgebildete Schiebeschaftinstrumente bekannt, die unter anderem als Bandscheibenzange oder auch bei Operationen zur Verkleinerung von Nasenmuscheln eingesetzt werden. Wie bei Laminektomie Rongeuren besteht auch bei Conchotomen das Problem einer nur unzureichenden Reinigbarkeit.

Aus der US 2004/0044346 A1 ist ein chirurgisches Instrument bekannt, bei welchem ein vorderer Abschnitt eines Schiebeteils um eine senkrecht zur Längserstreckung des Schaftes verlaufende Drehachse verschwenkbar ist, wobei die Schiebeteilspitze in einer seitlich verschwenkten Position ohne Weiteres von einem Rückabschnitt des Schiebeteils abnehmbar ist bzw. sich unmittelbar löst. Nachteilig bei dem bekannten chirurgischen Instrument ist die notwendige Re-Montage nach erfolgter Reinigung.

Der Erfindung liegt die Aufgabe zugrunde, ein alternatives, gut reinigbares Schiebeschaftinstrument anzugeben. Bevorzugt soll das Schiebeteil mittels Einhandbedienung in eine Reinigungsstellung überführbar sein. Weiter bevorzugt soll sich das chirurgische Instrument durch einen einfachen Aufbau auszeichnen.

Diese Aufgabe wird mit einem chirurgischen Instrument (Schiebeschaftinstrument) mit den Merkmalen des Anspruchs 1 gelöst. Vorteilhafte Weiterbildungen der Erfindung sind in den Unteransprüchen angegeben. In den Rahmen der Erfindung fallen sämtliche Kombinationen aus zumindest zwei von in der Beschreibung, den Ansprüchen und/oder den Figuren offenbarten Merkmalen.

Der Erfindung hat erkannt, dass es als Grundvoraussetzung zur Lösung sämtlicher vorgenannter Aufgaben notwendig ist, von der bisher üblichen Konstruktion abzuweichen und die Drehachse, um die das Schiebeteil in seiner Reinigungsstellung verschwenkbar ist, nicht wie im Stand der Technik gleichzeitig senkrecht zur Längserstreckung des Schaftes sowie senkrecht zur Längserstreckung des verschwenkbaren Griffteils anzuordnen, sondern derart, dass das Schiebeteil in einer Drehebene verschwenkbar ist, die senkrecht verläuft zu einer von dem Schaftteil und dem verschwenkbaren Griffteil aufgespannten Ebene. Anders ausgedrückt verläuft die Drehachse, um die das Schiebeteil eines erfindungsgemäßen chirurgischen Instrumentes in seiner Reinigungsstellung verschwenkbar ist, nicht wie im Stand der Technik parallel zur Schwenkachse, um die das verschwenkbare Griffteil beim Betätigen verschwenkbar ist, sondern liegt vielmehr in einer gedachten, vom Griffteil und dem Schaftteil aufgespannten Ebene, verläuft also sowohl senkrecht zur Längserstreckung des Schaftteils als auch senkrecht zur Schwenkachse des verschwenkbaren Griffteils. Diese völlig neue Konstruktionsweise gewährleistet erstmals einen einfachen und gut reinigbaren Aufbau des chirurgischen Instrumentes und ermöglicht überraschender Weise durch später noch zu erläuternde zusätzliche Maßnahmen eine Einhandüberführbarkeit des Schiebeteils aus der Arbeitsstellung in seine Reinigungsstellung.

ten Ebene, verläuft also sowohl senkrecht zur Längserstreckung des Schaftteils als auch senkrecht zur Schwenkachse des verschwenkbaren Griffteils. Diese völlig neue Konstruktionsweise gewährleistet erstmals einen einfachen und gut reinigbaren Aufbau des chirurgischen Instrumentes und ermöglicht überraschender Weise durch später noch zu erläuternde zusätzliche Maßnahmen eine Einhandüberführbarkeit des Schiebeteils aus der Arbeitsstellung in seine Reinigungsstellung.

Ganz besonders bevorzugt ist eine Ausführungsform des chirurgischen Instrumentes, bei der das Schiebeteil zum Reinigen der Schaftoberseite und der Schiebeteilunterseite nicht von dem Schaft entfernt werden muss, sondern vielmehr auch nach dem Verschwenken um die Drehachse unverlierbar am Schaft gehalten ist. Hierdurch entfällt die im Stand der Technik häufig auftretende Notwendigkeit der Re-Montage des chirurgischen Instrumentes nach einem Reinigungsvorgang. Um das Schiebeteil unverlierbar am Schaftteil zu halten, ist es möglich, die Drehachse mittels eines Schraub- oder Nietstiftes zu bilden, der in einem Gegenelement gehalten (gefangen) ist.

In Weiterbildung der Erfindung ist mit Vorteil vorgesehen, dass dem Schiebeteil Verriegelungsmittel vorgesehen sind, die das Schiebeteil in der Arbeitsstellung, in der es nicht um die Drehachse verschwenkbar, jedoch längsverschieblich relativ zum Schaft geführt angeordnet ist, sichern, um ein unbeabsichtigtes Überführen in die Reinigungsstellung, in der das Schiebeteil dann um die Drehachse relativ zum Schaft verschwenkbar ist, sicher zu verhindern.

Ganz besonders bevorzugt sind die Verriegelungsmittel derart ausgebildet, dass diese bei Betätigung eine Anschlagposition für das mit dem Schiebeteil mechanisch gekoppelte (wirkverbundene) verschwenkbare Griffteil verstellend ausgebildet sind. Dabei definieren die unterschiedlichen Anschlagpositionen zum einen die Arbeitsstellung, in der das Schiebeteil nicht verschwenkbar längsverschieblich am Schaftteil geführt ist und zum anderen die Reinigungsstellung, in der das Schiebeteil aus der Führung befreit und um die Drehachse relativ zum Schaft verschwenkbar ist. Anders ausgedrückt ermöglichen die Verriegelungsmittel nach Betätigung ein im Vergleich zur Arbeitsstellung weiteres Verschwenken des Griffteils und damit ein weiteres Längsverschieben des Schiebeteils relativ zu dem Schaft (vorzugsweise nach hinten), so dass das Schiebeteil aus der Führung freikommt und dann in der Reinigungsstellung um die Drehachse verschwenkbar ist.

Ganz besonders bevorzugt ist eine Ausführungsform des chirurgischen Instrumentes, bei der die unterschiedlichen Anschlagpositionen für das verschwenkbare Griffteil von unterschiedlichen Durchmesserabschnitten eines Verriegelungsbolzens der Verriegelungsmittel gebildet werden, wobei sich die Durchmesserabschnitte hinsichtlich der Größe ihres Durchmessers unterscheiden. Bevorzugt weist der die Reinigungsstellung definierende Durchmesserabschnitt einen geringeren Durchmesser auf als der die Arbeitsstellung definierende Durchmesserabschnitt, um somit ein weiteres Verschwenken des verschwenkbaren Griffteils und damit ein weiteres Längsverschieben des Schiebeteils relativ zum Schaftteil zu ermöglichen.

Besonders zweckmäßig ist es, wenn der vorerwähnte, mindestens zwei unterschiedliche Durchmesserabschnitte aufweisende Verriegelungsbolzen federkraftbelastet ist, vorzugsweise derart, dass der Verriegelungsbolzen entgegen der Kraft der Feder in die die Reinigungsstellung definierende Entriegelungs- bzw. Entsperrposition verstellbar ist. Die Feder ist dann also bestrebt, die Verriegelungsmittel selbsttätig zu sperren und das Schiebeteil in seiner Arbeitsstellung zu sichern.

Das Vorsehen von Verriegelungsmitteln mit einem mindestens zwei, insbesondere axial benachbarte Durchmesserabschnitte aufweisenden Verriegelungsbolzen ermöglicht in Kombination mit der erfindungsgemäßen Ausrichtung der Drehachse für das Schiebeteil eine gute Einhandbedienbarkeit des chirurgischen Instrumentes.

Ganz besonders bevorzugt ist eine Ausführungsform des chirurgischen Instrumentes, bei der das verschwenkbare Griffteil gegen den mindestens zwei Durchmesserabschnitte aufweisenden Verriegelungsbolzen der Verriegelungsmittel, also in Richtung seiner Anschlagposition, federkraftbelastet ist, so dass das verschwenkbare Griffteil und damit das Schiebeteil nach Betätigen des Verriegelungsbolzens unmittelbar automatisch in die Reinigungsstellung verstellt wird, in der das Schiebeteil um die Drehachse relativ zum Schaft verschwenkbar ist. Eine derartige Ausführungsform ist optimal im Hinblick auf die Gewährleistung einer Einhandbedienbarkeit.

Besonders zweckmäßig ist es, wenn zur Federkraftbelastung des verschwenkbaren Griffteils eine, insbesondere zweiteilige, Spreizfeder vorgesehen ist, die zwischen dem verschwenkbaren Griffteil und einem, vorzugsweise einteilig mit dem Schaft ausgebildeten, Griffteil angeordnet ist, wobei es weiter bevorzugt ist, wenn sich die Spreizfeder an beiden vorgenannten Griffteiten abstützend angeordnet ist und bestrebt ist, die Griffteile auseinander zu drücken und damit das verschwenkbare Griffteil um die senkrecht zur Drehachse des Schiebeteils verlaufende Schwenkachse zu verschwenken.

Wie zuvor bereits angedeutet, ist es besonders bevorzugt, wenn der Verriegelungsbolzen der Verriegelungsmittel entgegen der Federkraft einer Feder aus seiner Sicherungsposition, in der der Verriegelungsbolzen das Schiebeteil in seiner Arbeitsstellung sichert, in eine Freigabeposition (Entsperrposition, Entsicherungsposition) überführbar ist, in der das Schiebeteil um die Drehachse verschwenkbar ist, wobei die Anschlagposition für das verschwenkbare Griffteil in der Freigabeposition gegenüber der Anschlagposition für das verschwenkbare Griffteil in der Sicherungsposition verstellt ist, vorzugsweise in proximale Richtung, also in eine Richtung weg von den distalen Arbeitsenden von Schiebeteil und Schaft.

Wie eingangs bereits erläutert, ist es besonders bevorzugt, wenn das chirurgische Instrument als in der Art einer Stanze arbeitender Rongeur ausgebildet ist, mittels dem große Kräfte im Bereich der distalen Arbeitsenden von Schaft und Schiebeteil aufgebracht werden können, um insbesondere Gewebe, Knorpel und/oder Knochen durchtrennen zu können. Bevorzugt wirken Schaft und Schiebeteil hierzu im Bereich ihrer distalen Arbeitsenden in der Art einer Stanze zusammen.

Neben einer Ausführungsform, bei der Schaft und Schiebeteil unmittelbar mit ihren digitalen Arbeitsenden, insbesondere in der Art einer Stanze, zusammenwirken, ist eine Ausführungsform realisierbar, bei der von dem Schiebeteil ein verschwenkbar angeordnetes Wirkorganteil (insbesondere Maulteil) betätigbar ist. Dabei arbeitet das Wirkorgan (insbesondere Maul) des chirurgischen Instrumentes bevorzugt in der Art einer Schere oder Zange. Ganz besonders bevorzugt ist eine Ausführungsform des chirurgischen Instrumentes als Conchotom, insbesondere zum Einsatz als Bandscheibenzange oder bei Hals-, Nasen-, Ohrenoperationen, wobei sich ein Conchotom durch ein zangenartiges Maul mit mindestens einem, vorzugsweise ausschließlich einem, verschwenkbar angeordneten Maulteil auszeichnet.

Bevorzugt ist das Schiebeteil zum Betätigen des Maulteils mit Abstand zu einer Schwenkachse des Maulteils mit dem Maulteils in Eingriff. Anders ausgedrückt ist das Schiebeteil mit Abstand zur Schwenkachse des Maulteils gelenkig mit dem Maulteil zum Verschwenken desselben um die Schwenkachse gekoppelt.

Im Hinblick auf die konkrete Ausbildung der Kopplung des Schiebeteils mit dem Maulteil gibt es unterschiedliche Möglichkeiten. So ist es beispielsweise realisierbar, dass das Schiebeteil nicht unmittelbar, sondern mittelbar über mindestens ein Zwischenteil mit dem Maulteil mechanisch gekoppelt ist. Ganz besonders bevorzugt ist jedoch eine direkte Kopplung von Schiebeteil und Maulteil, vorzugsweise derart, dass am Maulteil ein, vorzugsweise endseitig offener Aufnahmeschlitz realisiert ist, in den das Schiebeteil mit einem, vorzugsweise stab- bzw. stangenförmigen Mitnehmerabschnitt eingreift. Durch Längsverstellen des Schiebeteils wird dann das Maulteil um seine Schwenkachse verschwenkt, wobei bei dieser Schwenkbewegung auch das Maulteil relativ zu dem Schiebeteil verschwenkt wird. Der Aufnahmeschlitz ist dabei mit Vorteil derart angeordnet und ausgeformt, dass diese Relativverstellbewegung zwischen Maulteil und Schiebeteil möglich wird. Ganz besonders bevorzugt verläuft der Aufnahmeschlitz nicht gerade, sondern ist schräg oder gebogen konturiert, wobei noch weiter bevorzugt ist, wenn der Aufnahmeschlitz (etwas nach vorne) bei geschlossenem Maul in Richtung des distalen Endes des chirurgischen Instrumentes gebogen ist.

Besonders bevorzugt ist eine Ausführungsform des chirurgischen Instrumentes, insbesondere des Conchotoms, bei der der Aufnahmeschlitz eine Demontage des chirurgischen Instrumentes zu Reinigungszwecken erleichternd ausgebildet ist. Bevorzugt ist der Aufnahmeschlitz hierzu derart konturiert, dass wenn die Verriegelungseinrichtung des chirurgischen Instrumentes entriegelt ist und das Maulteil unmittelbar manuell betätigt wird der Mitnehmerabschnitt des Schiebeteils aus dem Aufnahmeschlitz heraus verstellt wird, um das Schiebeteil danach um die Drehachse zu Reinigungszwecken verschwenken zu können. Bevorzugt ist der Aufnahmeschlitz hierzu in der Art einer Führungskulisse gebogen konturiert.

Insbesondere bei einer wie zuvor beschrieben realisierten Ausgestaltung des Aufnahmeschlitzes ist es bevorzugt, wenn der, insbesondere stab- bzw. stangenförmige Mitnehmerabschnitt an beiden Endseiten gestützt ist, indem der Mitnehmerabschnitt zwischen zwei Seitenabschnitten des Schiebeschaftes aufgenommen. Vorzugsweise ist der Mitnehmerabschnitt einteilig mit den beiden Seitenabschnitten des Schiebeschaftes ausgebildet, wobei es alternativ realisierbar ist, den Mitnehmerabschnitt als separates Bauteil auszubilden und zwischen den Seitenabschnitten des Schiebeschaftes, beispielsweise durch Verschweißen oder Aufnahme in zwei Fixieröffnungen festzulegen. Bei einer alternativen Ausführungsform ist der Mitnehmerabschnitt nur einseitig fixiert und weist ein freies Ende auf. Bei einer derartigen Ausführungsform kann der Mitnehmerabschnitt einfach durch seitliches Verschwenken des Schiebeteils aus dem Aufnahmeschlitz des Maulteils herausgeführt werden. Eine derartige Ausführungsform eignet sich insbesondere für vergleichsweise große chirurgische Instrumente, bei denen der Mitnehmerabschnitt aus Stabilitätsgründen eine ausreichende Dickenstreckung aufweist.

Im Hinblick auf die Ausgestaltung der Führung, die das Schiebeteil beim Betätigen des verschwenkbaren Griffteils entlang des Schaftes führt gibt es unterschiedliche Möglichkeiten.. Besonders bevorzugt ist eine Ausgestaltung der Führung als vorzugsweise im Schaft vorgesehene und oben offene, in Querschnitt T-Nut. Besonders bevorzugt greift das Schiebeteil mit einem im Querschnitt entsprechend T-förmig ausgeformten Führungsfortsatz in die T-Nut ein, wobei der Schiebeschaft bei entriegelten, d.h. in der Freigabeposition befindlichen, Verriegelungsmitteln aus der T-Nut, insbesondere nach oben, freikommen kann und ansonsten in der Führung axial verschieblich gefangen ist.

Grundsätzlich ist eine Ausführungsform realisierbar, bei der das Schiebeteil bei in der Freigabeposition befindlichen (entriegelten) Verriegelungsmitteln unmittelbar seitlich aus der Führung freikommen kann, wobei hierzu bevorzugt eine seitliche Aussparung im Schaft realisiert ist. Besonders bevorzugt ist jedoch eine Ausführungsform, insbesondere bei der Ausbildung des chirurgischen Instrumentes als Conchotom, bei der das Schiebeteil, vorzugsweise durch Eigenspannung, in eine Richtung von dem Schaft weg vorgespannt ist, so dass das Schiebeteil bei entriegelten Verriegelungsmitteln und bei einer Entkopplung vom Maulteil sich selbsttätig in eine Position verstellt, in der es um die Drehachse verschwenkbar ist. Besonders bevorzugt ist das Schiebeteil zum Realisieren der Eigenspannung (leicht) gekrümmt ausgeformt und in einem in der Führung geführten Zustand elastisch verformt, so dass das Schiebeteil, solange dieses längsverschieblich geführt ist, vorgespannt ist, um dann bei entriegelten Verriegelungsmitteln von dem Schaft weg, vorzugsweise in Richtung der Längserstreckung der Drehachse von dem Schaft weg, also bevorzugt nach oben, zu verschwenken bzw. zu federn, um daraufhin um die Drehachse verschwenkt zu werden.

Die Erfindung führt auch auf ein, als eigenständige Erfindung offenbartes und beanspruchbares chirurgisches Instrument, das sich durch optimierte Verriegelungsmittel auszeichnet, die das Schiebeteil gegen ein unbeabsichtigtes Überführen in die Reinigungsstellung sichern. Diese Verriegelungsmittel wurden zuvor bereits im Zusammenhang mit einer senkrecht zur Schwenkachse des verschwenkbaren Griffteils verlaufenden Drehachse beschrieben, wobei die nach dem Konzept der Erfindung ausgebildeten Verriegelungsmittel auch mit anders orientierten, beispielsweise parallel zur Schwenkachse verlaufenden Drehachsen für das Schiebeteil realisierbar sind. Die Verriegelungsmittel zeichnen sich dadurch aus, dass sie einen Anschlag für das verschwenkbare Griffteil des chirurgischen Instrumentes bilden, wobei zwei unterschiedliche Durchmesserabschnitte eines Verriegelungsbolzens der Verriegelungsmittel zwei unterschiedliche Anschlagpositionen für das verschwenkbare Griffteil definieren, wobei eine der Anschlagpositionen die Arbeitsstellung und die andere Anschlagsposition die Reinigungsstellung des längsverschieblichen und um die Drehachse verschwenkbaren Schiebeteils definieren. Ganz besonders bevorzugt ist eine Ausführungsform des chirurgischen Instrumentes, bei der das verschwenkbare Griffteil gegen den Verriegelungsbolzen der Verriegelungsmittel federkraftbeaufschlagt ist.

Die Erfindung betrifft also ein Chirurgisches Instrument, insbesondere Laminektomie Rongeur oder Conchotom, insbesondere nach einem der vorhergehenden Ansprüche, mit einem Schaft und mit einem relativ zum Schaft längsverschiebbaren Schiebeteil, welchem zum Längsverschieben ein um eine Schwenkachse verschwenkbares Griffteil zugeordnet ist, wobei das Schiebeteil aus einer Arbeitsstellung, in der das Schiebteil an dem Schaft in einer Führung längsverschiebbar geführt ist, in eine Reinigungsstellung überführbar ist, in der das Schiebeteil aus der Führung befreit ist und relativ zum Schaft um eine senkrecht zur Längserstreckung des Schaftes orientierte Drehachse verschwenkbar ist, wobei Verriegelungsmittel vorgesehen sind, mit denen das Schiebeteil in der Arbeitsstellung gegen ein unbeabsichtigtes Überführen in die Reinigungsstellung sicherbar ist, wobei die Verriegelungsmittel einen, vorzugsweise senkrecht zur Drehachse, verstellbaren Verriegelungsbolzen mit einem die Anschlagposition für das verschwenkbare Griffteil in der Arbeitsstellung definierenden ersten Durchmesserabschnitt und einen die Anschlagposition für das verschwenkbare Griffteil in der Reinigungsstellung definierenden zweiten Durchmesserabschnitt aufweist. In Weiterbildung der Erfindung ist vorteihafterweise vorgesehen, dass die Verriegelungsmittel eine Anschlagsposition für das verschwenkbare Griffteil verstellend ausgebildet sind. In Weiterbildung der Erfindung ist vorteilhafterweise vorgesehen, dass der Durchmesser des ersten Durchmesserabschnitts größer ist als der Durchmesser des zweiten Durchmesserabschnitts. In Weiterbildung der Erfindung ist vorteilhafterweise vorgesehen, dass das verschwenkbare Griffteil gegen den Verriegelungsbolzen federkraftbelastet ist.

Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:
- Fig. 1:: eine (teilgeschnittene) Seitenansicht eines als Schiebeschaf- tinstrument ausgebildeten chirurgischen Instrumentes, wobei sich das Schiebeteil in einer Arbeitsstellung befindet, in der das Schiebeteil längsverschieblich zum Schaft geführt ist,
- Fig. 2:: eine Draufsicht auf das chirurgische Instrument gemäß Fig. 1, wobei sich das Schiebeteil in einer Reinigungsstellung befindet, in der das Schiebeteil (wie gezeigt), um eine Drehachse relativ zum Schaft verschwenkbar ist, wobei sich die Drehachse sowohl senkrecht zur Längserstreckung des Schaftes als auch senk- recht zu einer Schwenkachse erstreckt, die ein vorderes, verschwenkbares Griffteil des chirurgischen Instrumentes ist,
- Fig. 3:: eine vergrößerte, teilgeschnittene Detaildarstellung von Verrie- gelungsmitteln zum Sichern des Schiebeteils in seiner Arbeits- stellung,
- Fig. 4:: ein demontiertes Schiebeteil,
- Fig. 5:: einen Schaft des chirurgischen Instrumentes mit integralem fest- stehendem Griffteil,
- Fig. 6:: ein verschwenkbares Griffteil,
- Fig. 7:: eine Explosionsdarstellung der Drehachsenkonstruktion zum unverlierbaren Halten des Schiebeteils am Schaft,
- Fig. 8:: eine Darstellung des verschwenkbaren Griffteils in seiner entrie- gelten Stellung, in der das Schiebeteil in die Reinigungsstellung überführt ist,
- Fig. 9:: eine Darstellung des verschwenkbaren Griffteils in seiner Siche- rungsposition, in der das Schiebeteil gegen unbeabsichtigtes Überführen in die Reinigungsposition gesichert ist,
- Fig. 10a:: in als Conchotom ausgebildetes chirurgisches Instrument mit geöffnetem Maul (Wirkorgan),
- Fig. 10b:: eine vergrößerte Darstellung des geöffneten Mauls aus Fig. 10a,
- Fig. 11a:: ein als Conchotom ausgebildetes chirurgisches Instrument mit geschlossenem Maul,
- Fig. 11b:: eine vergrößerte Darstellung des geschlossenen Mauls aus Fig. 11a, und
- Fig. 12:: eine Draufsicht auf ein als Conchotom ausgebildetes chirurgi- sches Instrument mit um eine in Hochrichtung verlaufende Drehachse verschwenktem Schiebeteil.

In den Figuren sind gleiche Elemente und Elemente mit der gleichen Funktion mit den gleichen Bezugszeichen gekennzeichnet.

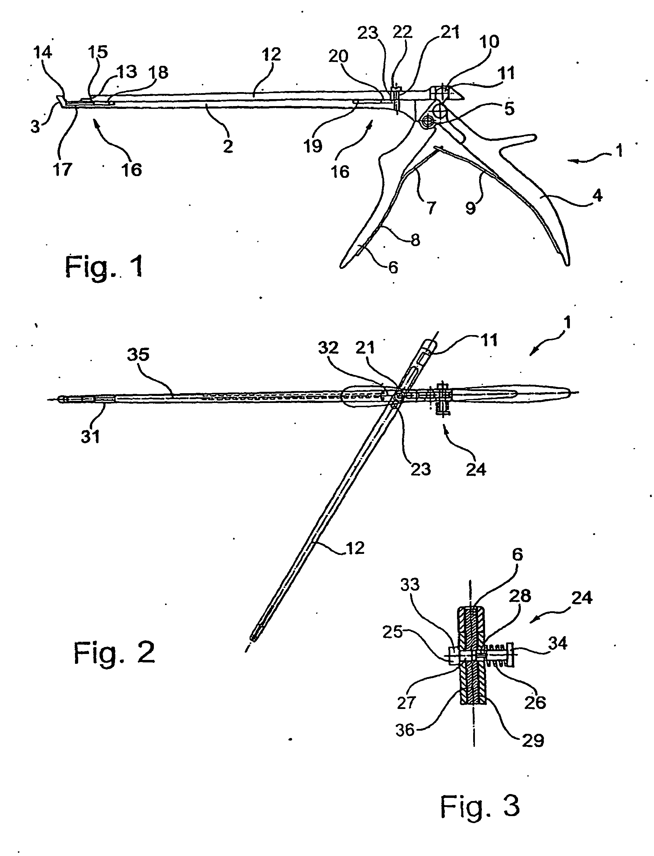

In den Fig. 1 und 2 ist ein als Stanze dienendes chirurgisches Instrument 1 gezeigt. Das chirurgische Instrument 1 umfasst einen langgestreckten Schaft 2 mit einem distalen (vorderen) Arbeitsende 3. Einteilig mit dem Schaft 2 ist ein hinteres Griffteil 4 (Griffbranche) ausgebildet.

Relativ zu dem feststehenden, proximalen (hinteren) Griffteil 4 ist um eine quer zur Längserstreckung des Schaftes 2 verlaufende Schwenkachse 5 ein distales (vorderes) verschwenkbares Griffteil 6 verschwenkbar angeordnet. Zwischen beiden Griffteilen 4, 6 ist in an sich bekannter Weise eine Spreizfeder 7 angeordnet, die in dem gezeigten Ausführungsbeispiel aus zwei Blattfederteilen 9 gebildet ist, wobei jedes Blattfederteil 8, 9 mit einem Griffteil 6, 4 verschraubt ist und wobei die Blattfederteile 8, 9 mit ihren freien Enden ineinander greifen.

Das verschwenkbare (vordere) Griffteil 6 weist ein oberes, näherungsweise kugelkopfförmiges, Ende 10 auf, welches in eine seitlich offene Aussparung 11 eines Schiebeteils 12 eingreift. Auf diese Weise ist das Schiebeteil 12 mittels des vorderen, verschwenkbaren Griffteils 6, durch Verschwenken desselben um die Schwenkachse 5 relativ zu dem Schaft 2 längsverschieblich.

Das Schiebeteil 12 weist ein distales (vorderes) Arbeitsende 13 auf, das mit dem vorderen Arbeitsende 3 des Schaftes 2 in der Art einer Stanze zusammenwirkt. Hierzu weisen die Arbeitsenden 3, 13 einander zugewandte Schneidflächen 14, 15 (Stanzflächen) auf.

In Fig. 1 ist das Schiebeteil 12 in einer Arbeitsstellung gezeigt. In der Arbeitsstellung ist das Schiebeteil 12 längsverschieblich am Schaft 2 geführt. Als Führung 16 ist im Bereich des distalen Endbereiches des Schaftes 2 eine Führungsschiene 17 vorgesehen, in die ein unterer Führungsfortsatz 18 im Bereich des distalen Endbereichs des Schiebeteils 12 eingreift. Ferner umfasst die Führung 16 in einem proximal zu den Arbeitsenden 3, 13 beabstandeten Bereich eine Führungsschiene 19 am Schaft 2, welche ebenfalls mit einem unteren Führungsfortsatz 20 des Schiebeteils 12 zusammenwirkt. Die Führung 16 ist so konzipiert, dass das Schiebeteil 12 in seiner Arbeitsstellung über den gesamten Längsverschiebeweg entlang des Schaftes, also auch in einer distal ausgelenkten und in einer proximal ausgelenkten Endposition in der Führung 16 gefangen ist.

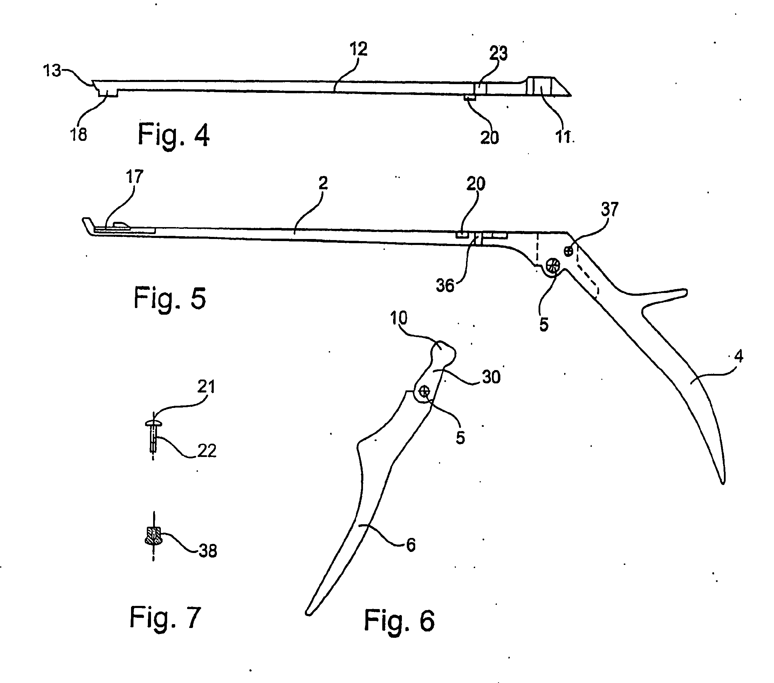

Um eine erleichterte Reinigung des chirurgischen Instrumentes 1 zu ermöglichen, ist eine Drehachse 21 für das Schiebeteil 12 vorgesehen. Ein Verschwenken um diese Drehachse 21 ist in der gezeigten Arbeitsstellung, in der das Schiebeteil 12 in der Führung 16 gefangen ist, jedoch nicht möglich. Wie sich aus Fig. 1 ergibt, durchsetzt die von einer Kopfschraube 22 gebildete Drehachse 21 ein Langloch 23 im Schiebeteil 12, so dass das Schiebeteil 12 relativ zur Drehachse 21 längsverschieblich ist. Die Kopfschraube 22 durchsetzt weiterhin den Schaft 2 und ist, wie später noch erläutert werden wird, in einem Gegenelement (vgl. Fig. 7) gehalten (gefangen).

Dem Schiebeteil 12 sind in Fig. 3 dargestellte Verriegelungsmittel 24 zugeordnet, die das Schiebeteil 12 in der in Fig. 1 dargestellten Arbeitsstellung sichern. Die Verriegelungsmittel 24 umfassen einen Verriegelungsbolzen 25, der entgegen der Kraft einer als Schraubendruckfeder ausgebildeten Feder 26 aus der in Fig. 3 dargestellten, die Arbeitsstellung definierenden Sicherungsposition in eine Freigabeposition (Entsicherungs-, Entsperrposition) verstellbar ist. Der Verriegelungsbolzen 25 umfasst einen zylindrischen, ersten Durchmesserabschnitt 27, der eine erste Anschlagposition für das verschwenkbare Griffteil 6 definiert. Axial an den ersten Durchmesserabschnitt 27 grenzt ein durch Abdrehen hergestellter zweiter Durchmesserabschnitt 28 an, der eine Anschlagposition für das verschwenkbare Griffteil 6 in einer Reinigungsstellung des Schiebeteils 12 definiert. Anders ausgedrückt definiert der zweite Durchmesserabschnitt 28 einen Anschlag für das verschwenkbare Griffteil 6, welcher wiederum die Reinigungsstellung des Schiebeteils 12 definiert, in der das Schiebeteil 12 um die Drehachse 21 verschwenkbar ist. Wie sich aus Fig. 3 ergibt, durchsetzt der parallel zur Schwenkachse 5 und senkrecht zur Drehachse 21 orientierte Verriegelungsbolzen 25 das aus dem Schaft 2 und dem proximalen Griffteil 4 gebildete Bauteil. Dabei durchragt der Verriegelungsbolzen 25 eine innere Ausnehmung 29, in der ein Abschnitt 30 des verschwenkbaren Griffteils 6 aufgenommen ist. Der Verriegelungsbolzen 25 ist unverlierbar gehalten. Hierzu weist der Verriegelungsbolzen 25 radial verbreiterte Enden 33, 34 auf, die ein Herausschieben des Verriegelungsbolzens 25 aus der in aufnehmender Durchgangsöffnung sicher verhindern.

Die Verriegelungsmittel 24 sind besonders gut aus einer Zusammenschau der Fig. 3, 8 und 9 ersichtlich.

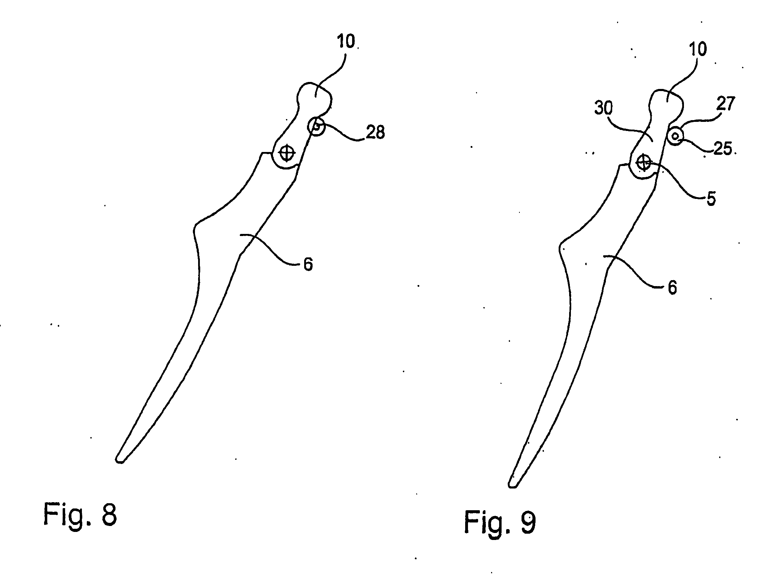

Wie erläutert, ist in Fig. 3 eine Sicherungsposition des Verriegelungsbolzens 25 gezeigt. Diese findet sich auch in Fig. 9 wieder. Zu erkennen ist, dass das verschwenkbare Griffteil 6 am ersten Durchmesserabschnitt 27 anliegt, also eine Anschlagposition für das verschwenkbare Griffteil 6 relativ weit distal liegt. Zum proximalen Verschieben der Anschlagposition für das verschwenkbare Griffteil 6, wird der Verriegelungsbolzen 25 entgegen der Federkraft der Feder 26 betätigt, so dass der zweite, reduzierte Durchmesserabschnitt 28 in dem Bereich innerhalb der inneren Ausnehmung 29 zum Liegen kommt und das verschwenkbare Griffteil 6 mit dem zweiten Durchmesserabschnitt 28 zusammenwirkt. In dieser Freigabeposition ist die Anschlagposition für das verschwenkbare Griffteil 6 nach proximal verschoben, so dass das Schiebeteil 12, welches über das obere Ende 10 mit dem verschwenkbaren Griffteil 6 gekoppelt ist, weiter nach hinten, also proximal verschoben werden kann. In dieser nach hinten verschobenen Stellung (Reinigungsstellung) ist das Schiebeteil 12 aus der Führung 16 frei, d.h. der Führungsfortsatz 18 ist nicht mehr in Eingriff mit der Führungsschiene 17 und der Führungsfortsatz 20 ist nicht mehr in Eingriff mit der Führungsschiene 19, sondern die Führungsfortsätze 18, 20 können durch seitliche Ausnehmungen 31, 32 hinausverschwenkt werden. Dadurch, dass in der Freigabeposition das Schiebeteil 12 nicht mehr in der Führung 16 gefangen ist, kann das Schiebeteil 12 um die Drehachse 21, also in einer Horizontalebene verschwenkt werden.

Eine optimale Einhandbedienbarkeit des chirurgischen Instrumentes 1 bzw. der Verriegelungsmittel 24 ist deshalb gegeben, weil das verschwenkbare Griffteil 6 über die Spreizfeder 7 immer in Richtung der jeweiligen Anschlagposition, d.h. in Richtung Verriegelungsbolzen 25, federkraftbeaufschlagt ist, so dass das verschwenkbare Griffteil 6 automatisch nach Betätigen des Verriegelungsbolzens 25 mit seinem oberen Bereich proximal, d.h. nach hinten, verschwenkt und somit in die Freigabeposition einschnappt, die gleichzeitig die Reinigungsstellung des Schiebeteils 12 definiert.

Fig. 2 zeigt das chirurgische Instrument 1 mit in der Reinigungsstellung verschwenktem Schiebeteil 12. Zu erkennen ist, dass in diesem verschwenkten Zustand eine Schaftoberseite 35, die ansonsten von dem Schiebeteil 12 verdeckt ist, optimal reinigbar ist. Das gleiche gilt für eine in Fig. 2 nicht zu erkennende, der Schaftoberseite 35 zugewandte Schaftunterseite.

In den Fig. 4 bis 7 sind verschiedene Einzelteile des chirurgischen Instrumentes 1 dargestellt, wobei hier nochmals zu bemerken ist, dass das chirurgische Instrument 1 in der Praxis nicht in diese Einzelteile zerlegt werden muss, sondern im montierten Zustand optimal reinigbar ist.

Zu erkennen ist in Fig. 4 das langgestreckte Schiebeteil 12 mit seinem distalen unteren Fortsatz 18 sowie mit seinem proximalen unteren Führungsfortsatz 20, wobei die Führungsfortsätze 18, 20 Teil der Führung 16 sind. Zu erkennen ist ferner das Langloch 23, das üblicherweise von der Kopfschraube 22 (vgl. Fig. 7) durchsetzt ist, und welches ein Relativverschieben des Schiebeteils 12 relativ zur Drehachse 21 gewährleistet. Des Weiteren ist das distale Arbeitsende 13 zu erkennen, sowie die im proximalen Bereich befindliche seitlich offene Aussparung 11, die ein Entkoppeln des Schiebeteils 12 von dem verschwenkbaren Griffteil 6 durch Verschwenken des Schiebeteils 12 um die Drehachse 21 ermöglicht.

Fig. 5 zeigt das aus Schaft 2 und proximalem Griffteil 4 gebildete Bauteil. Dieses weist die anhand von Fig. 1 bereits erläuterten Führungsschienen 17, 20 auf, sowie eine Durchgangsbohrung 36 zur Aufnahme der Kopfschraube 22 (vgl. Fig. 7). Zu erkennen ist ferner eine Durchgangsöffnung 37, die im montierten Zustand vom nicht dargestellten Verriegelungsbolzen 25 durchsetzt ist, sowie die Position einer Schwenkachse 5 für das in Fig. 6 dargestellte verschwenkbare Griffteil 6.

Fig. 7 zeigt eine Möglichkeit zur unverlierbaren Halterung des Schiebeteils 12 am Schaft 2. Zu erkennen ist die die Drehachse 21 bildende Kopfschraube 22, die in einem Gegenelement 38 mit Innengewinde gehalten ist. Eine Demontage ist zwar theoretisch möglich, jedoch nicht notwendig. Es ist auch eine Ausführungsform realisierbar, bei der das hier als Kopfschraube 22 ausgebildete, die Drehachse 21 bildende Bauteil unlösbar in einem Gegenelement gehalten ist, oder bei Verzicht auf ein Gegenelement, beispielsweise durch Verstemmen am Schaft 2 festgelegt ist.

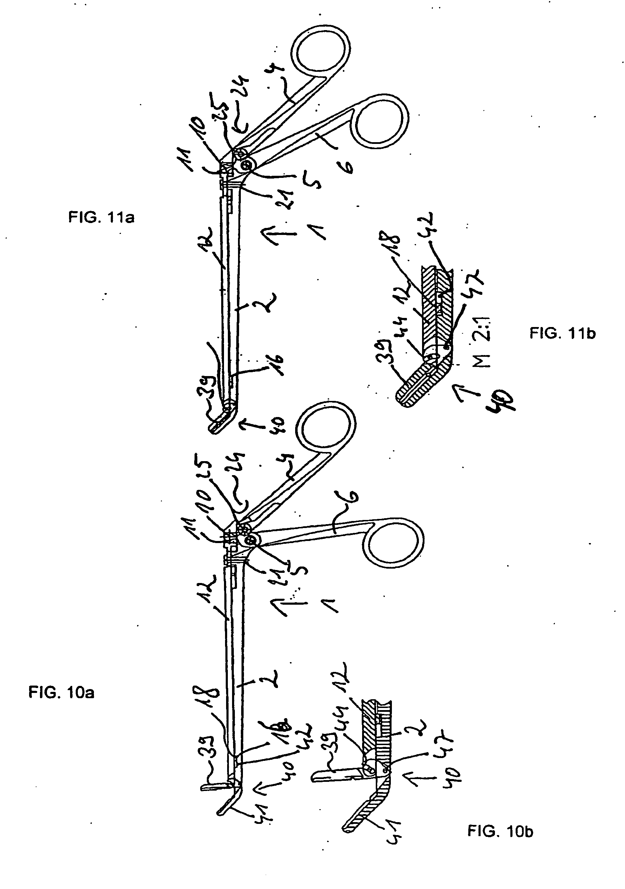

Im Folgenden wird anhand der Fig. 10a bis 12 ein alternatives, als Conchotom ausgebildetes chirurgisches Instrument 1 beschrieben. Das Conchotom ist mit den in den Fig. 3, 6, 7, 8 und 9 gezeigten Teilen bzw. Funktionseinheiten ausgestattet, die zur Vermeidung von Wiederholungen im Folgenden nicht nochmals erläutert werden. Bezüglich der Funktionsweise und des Aufbaus dieser Teile wird auf die entsprechenden vorangehenden Textstellen verwiesen.

Das als Conchotom ausgebildete chirurgische Instrument 1 umfasst ein langgestrecktes Schiebeteil 12, welches mit seinem distalen (vorderen) Ende in Eingriff ist mit einem verschwenkbar am Schaft 2 angelenkten Maulteil 39 (Wirkorganteil) eines distalen Mauls 40 (Wirkorgan) des chirurgischen Instrumentes 1. Das verschwenkbar angeordnete Maulteil 39 wirkt, wie insbesondere aus den vergrößerten Darstellungen gemäß den Fig. 10b und 11b hervorgeht, mit einem weiteren, hier feststehenden Maulteil 41 zusammen, welches einteilig ausgebildet ist mit dem Schaft 2 des chirurgischen Instrumentes 1. Einteilig mit dem Schaft 2 ist ebenso ein hinteres Griffteil 4 (Griffbranche) ausgeführt.

Relativ zu dem feststehenden, proximalen (hinteren) Griffteil 4 ist um eine quer zur Längserstreckung des Schaftes 2 verlaufende Schwenkachse 5 ein distales (vorderes) verschwenkbares Griffteil 6 verschwenkbar angeordnet. Zwischen beiden Griffteilen 4, 6 ist in dem gezeigten Ausführungsbeispiel im Gegensatz zu dem zuvor beschriebenen Laminektomie Rongeur keine Spreizfeder angeordnet, wobei grundsätzlich auch eine Ausbildung des Conchotoms mit einer Spreizfeder realisierbar ist.

Bis auf die Spreizfeder ist das Griff- und Verriegelungskonzept des Conchotoms identisch mit dem Griff- und Verriegelungskonzept des zuvor beschriebenen Laminektomie Rongeurs. So weist das (vordere) Griffteil ein oberes, näherungsweise kugelförmiges Ende 10 auf, welches in eine seitlich offene Aussparung 11 des Schiebeteils 12 eingreift. Auf diese Weise ist das Schiebeteil 12 mittels des vorderen, verschwenkbaren Griffteils 6, durch Verschwenken desselben um die Schwenkachse 5 relativ zu dem Schaft 2 längsverschieblich.

In den Fig. 10a bis 11b ist das Schiebeteil 12 in einer Arbeitsstellung gezeigt, wobei die Fig. 10a, 10b einerseits und die Fig. 11a und 11b andererseits axiale Extrempositionen des Schiebeteils 12 innerhalb der Arbeitsstellung zeigen. In den Fig. 10a und 10b ist das Schiebeteil 2 in seiner proximalen Endpositionen der Arbeitsstellung gezeigt, in der das Maul 40 maximal geöffnet ist, wohingegen in den Fig. 11a und 11b distale Endposition des Schiebeteils 12 gezeigt ist, in der das Maul 40 geschlossen ist, also das verschwenkbare Maulteil 39 am feststehenden Maulteil 41 anliegt. In der Arbeitsstellung ist das Schiebeteil 12 beim axialen Verschieben zwischen beiden vorher genannten maximalen Auslenkpositionen am Schaft geführt. Als Führung 16 ist eine im Querschnitt T-förmige Nut (T-Nut 42) im Schaft 2 vorgesehen, in die das Schiebeteil 12 in der Arbeitsstellung mit einem im Wesentlichen formkongruenten im Querschnitt T-förmigen unteren Führungsfortsatz 18 eingreift, wobei die T-Form in einer Ansicht (nicht gezeigt) von vorne betrachtet auf dem Kopf steht.

Um eine erleichterte Reinigung des chirurgischen Instrumentes 1 zu ermöglichen, ist eine sich in Hochrichtung erstreckende Drehachse 21 für das Schiebeteil 12 vorgesehen. Ein Verschwenken um diese Drehachse 21 ist in der gezeigten Arbeitsstellung (vgl. Fig. 10a bis 11b), in der das Schiebeteil 12 in der T-Nut gefangen ist, nicht möglich.

Dem Schiebeteil 12 sind in Fig. 3 gezeigte und in Zusammenhang mit Fig. 3 im Detail erläuterte Verriegelungsmittel 24 zugeordnet, die das Schiebeteil 12, wie zuvor beschrieben, in seiner Arbeitsstellung gegen ein unbeabsichtigtes Überführen in die Reinigungsstellung sichern. Die Verriegelungsmittel 24 und deren Funktionsweise sind besonders gut aus einer Zusammenschau der Fig. 3, 8 und 9 ersichtlich.

Die Fig. 10a und 11a zeigen den Verriegelungsbolzen 25 der Verriegelungsmittel 24 in einer Sicherungsposition. Wenn die Verriegelungsmittel 24 durch Verstellen des Verriegelungsbolzens 25 entgegen der Federkraft der Feder 26 entriegelt, eine Freigabeposition verstellt wird, liegt das vordere Griffteil 6 am zweiten (reduzierten) Durchmesserabschnitt des Verriegelungsbolzens 25 an. In dieser Freigabeposition (entsprechende Verriegelungsmittel 24) ist die Anschlagposition für das verschwenkbare Griffteil 6 nach proximal, also nach hinten, verschoben, so dass das Schiebeteil, welches über das obere Ende 10 mit dem verschwenkbaren Griffteil 6 gekoppelt ist, weiter nach hinten, also nach proximal, verschoben werden kann. Nach Lösen des Schiebeteils 12 von dem verschwenkbar angeordneten Maulteil 39 kann das Schiebeteil in der Zeichnungsebene nach oben von dem Schaft 2 weg in seine Reinigungsstellung federn, in der das Schiebeteil 12 um die Drehachse 21 zu Reinigungszwecken verschwenkbar ist. Um dieses Herausfedern aus der Führung 16 zu ermöglichen, ist das Schiebeteil 12 vor dem Einbau leicht mit seinem distalen Ende nach oben gebogen, weist also eine im unbelasteten Zustand gebogene Grundform auf und ist, wenn es sich in der Führung 16 befindet, nach oben, d.h. von dem Schaft 2 weg, durch Eigenspannung vorgespannt.

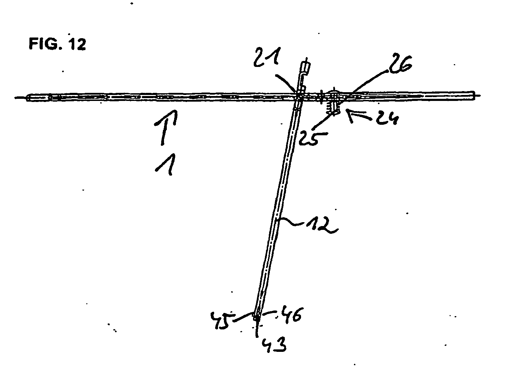

In den Fig. 10b und 11b ist das Maul 40 bzw. ist die Kopplung zwischen Schiebeteil 12 und verschwenkbarem Maulteil 39 im Detail gezeigt. Zu erkennen ist, dass das Schiebeteil 12 mit einem quer zu seiner Längserstreckung verlaufenden, stabförmigen Mitnehmerabschnitt in einen gebogenen, einseitig offenen Aufnahmeschlitz 44 des Maulteils 39 eingreift. Zu erkennen ist, dass der Aufnahmeschlitz 44 bei geschlossenem Maul 40 leicht nach vorne gebogen konturiert ist. Wie sich weiter aus den Fig. 10b und 11b ergibt, ist der Aufnahmeschlitz 44 mit Abstand zur Schwenkachse 47 des Maulteils 39 angeordnet, über die das Maulteil 39 am Schaft 2 fixiert ist. Der Mitnehmerabschnitt 43 ist, wie sich insbesondere aus Fig. 12 ergibt, zwischen zwei parallelen, distalen Seitenabschnitten 45, 46 aufgenommen, weist also kein freies Ende auf, sondern ist mit beiden Enden einteilig mit den Seitenabschnitten 45, 46 ausgebildet, oder alternativ als separates Bauteil an beiden Seitenabschnitten 45, 46, bevorzugt in entsprechenden seitlichen Aufnahmebohrungen der Seitenabschnitte 45, 46 gehalten. Eine derartige Anordnung des Mitnehmerabschnittes 43 ist insbesondere bei kleinen Baugrößen sinnvoll, um ein Tordieren des Schiebeteils 12 unter Last beim Betätigen des Griffteils 6 zu vermeiden. Insbesondere bei größeren Bauformen kann auf einen der beiden Seitenabschnitte 45, 46 verzichtet werden, so dass der Mitnehmerabschnitt 43 nur einseitig gehalten und dadurch ein seitliches Herausverschwenken aus dem Aufnahmeschlitz 44 ermöglicht ist. Bei einer derartigen Ausführungsform kann die Führung 16 beispielsweise in der Art wie bei dem Laminektomie Rongeur ausgebildet werden, aus der das Schiebeteil 12 seitlich in der Reinigungsposition heraus verschwenkt werden kann. Bei der gezeigten Ausführungsvariante muss das Schiebeteil 12 - hier durch Vorspannung - mit seinem distalen Ende erst nach oben von dem Schaft 2 weg bewegt werden.

Nach Überführen der Verriegelungsmittel 24 in die Freigabestellung (Freigabeposition) wird das Schiebeteil 12, wenn das Maulteil 39 manuell betätigt wird aus dem Aufnahmeschlitz 44 herausgeführt und kann nach oben von dem Schaft 2 weg aufgrund seiner Vorspannung herausfedern und danach um die Drehachse 21 verschwenkt werden, wie dies in Fig. 12 gezeigt ist.

### Bezugszeichenliste

- 1: chirurgisches Instrument
- 2: Schaft
- 3: Arbeitsende
- 4: proximales Griffteil
- 5: Schwenkachse
- 6: verschwenkbares Griffteil
- 7: Spreizfeder
- 8: Blattfederteil
- 9: Blattfederteil
- 10: Ende
- 11: Aussparung
- 12: Schiebeteil
- 13: Arbeitsende
- 14: Schneidfläche
- 15: Schneidfläche
- 16: Führung
- 17: Führungsschiene
- 18: Führungsfortsatz
- 19: Führungsschiene
- 20: Führungsfortsatz
- 21: Drehachse
- 22: Kopfschraube
- 23: Langloch
- 24: Verriegelungsmittel
- 25: Verriegelungsbolzen
- 26: Feder
- 27: erster Durchmesserabschnitt
- 28: zweiter Durchmesserabschnitt
- 29: Ausnehmung
- 30: Abschnitt
- 31: Ausnehmung
- 32: Ausnehmung
- 33: Ende
- 34: Ende
- 35: Schaftoberseite
- 36: Durchgangsbohrung
- 37: Durchgangsöffnung
- 38: Gegenelement
- 39: Maulteil
- 40: Maul
- 41: Maulteil
- 42: T-Nut
- 43: Mitnehmerabschnitt
- 44: Aufnahmeschlitz
- 45: Seitenabschnitt
- 46: Seitenabschnitt
- 47: Schwenkachse

## Patentansprüche

1. Chirurgisches Instrument, insbesondere Laminektomie Rongeur oder Conchotom, mit einem Schaft (2) und mit einem relativ zum Schaft (2) längsverschiebbaren Schiebeteil (12), welchem zum Längsverschieben ein um eine Schwenkachse (5) verschwenkbares Griffteil (6) zugeordnet ist, wobei das Schiebeteil (12) aus einer Arbeitsstellung, in der das Schiebteil (12) an dem Schaft (2) in einer Führung (16) längsverschiebbar geführt ist, in eine Reinigungsstellung überführbar ist, in der das Schiebeteil (12) aus der Führung (16) befreit ist und relativ zum Schaft (2) um eine senkrecht zur Längserstreckung des Schaftes (2) orientierte Drehachse (21) verschwenkbar ist, wobei die Drehachse (21) senkrecht zur Schwenkachse (5) des verschwenkbaren Griffteils (6) ausgerichtet ist,
**dadurch gekennzeichnet,**
**dass** das Schiebeteil (12) in der Arbeitsstellung, während der Überführung von der Arbeitsstellung in die Reinigungsstellung und in der Reinigungsstellung unverlierbar am Schaft (2) gesichert ist.

2. Chirurgisches Instrument nach Anspruch 1,
**dadurch gekennzeichnet,**
**dass** Verriegelungsmittel (24) vorgesehen sind, mit denen das Schiebeteil (12) in der Arbeitsstellung gegen ein unbeabsichtigtes Überführen in die Reinigungsstellung sicherbar ist.

3. Chirurgisches Instrument nach Anspruch 2,
**dadurch gekennzeichnet,**
**dass** die Verriegelungsmittel (24) eine Anschlagsposition für das verschwenkbare Griffteil (6) verstellend ausgebildet sind.

4. Chirurgisches Instrument nach einem der Ansprüche 2 oder 3,
**dadurch gekennzeichnet,**
**dass** die Verriegelungsmittel (24) einen, vorzugsweise senkrecht zur Drehachse (21), verstellbaren Verriegelungsbolzen (25) mit einem die Anschlagposition für das verschwenkbare Griffteil (6) in der Arbeitsstellung definierenden ersten Durchmesserabschnitt (27) und einen die Anschlagposition für das verschwenkbare Griffteil (6) in der Reinigungsstellung definierenden zweiten Durchmesserabschnitt (28) aufweist.

5. Chirurgisches Instrument nach Anspruch 4,
**dadurch gekennzeichnet,**
**dass** der Durchmesser des ersten Durchmesseranschnitts (27) größer ist als der Durchmesser des zweiten Durchmesserabschnitts (28).

6. Chirurgisches Instrument nach einem der Ansprüche 4 oder 5,
**dadurch gekennzeichnet,**
**dass** das verschwenkbare Griffteil (6) gegen den Verriegelungsbolzen (25) federkraftbelastet ist.

7. Chirurgisches Instrument nach einem der Ansprüche 4 bis 6,
**dadurch gekennzeichnet,**
**dass** der Verriegelungsbolzen (25) entgegen der Federkraft einer Feder (26) aus seiner Sicherungsposition, in der der Verriegelungsbolzen (25) das Schiebeteil (12) in seiner Arbeitsstellung sichert in eine Freigabeposition überführbar ist, in der das Schiebeteil (12) um die Drehachse (21) verdrehbar ist und in der die Anschlagposition für das verschwenkbare Griffteil (6) gegenüber der Anschlagposition für das verschwenkbare Griffteil (6) in der Sicherungsposition verstellt ist.

8. Chirurgisches Instrument nach einem der vorhergehenden Ansprüche,
**dadurch gekennzeichnet,**
**dass** Schaft (2) und Schiebeteil (12) im Bereich ihrer distalen Arbeitsenden (3, 13) zusammenwirken und/oder dass das Schiebeteil (12) ein, insbesondere als Mauteil (39) ausgebildetes Wirkorganteil eines, vorzugsweise als Maul (40) ausgebildeten Wirkorgans, betätigend angeordnet ist.

9. Chirurgisches Instrument nach Anspruch 8,
**dadurch gekennzeichnet,**
**dass** das Schiebeteil (12) mit Abstand zu einer Schwenkachse (47) des Maulteils (39) mit dem Maulteil (39), vorzugsweise lösbar, in Eingriff ist.

10. Chirurgisches Instrument nach einem der Ansprüche 8 oder 9,
**dadurch gekennzeichnet,**
**dass** das Maulteil (39) mit einem, insbesondere schräg oder gebogen konturierten, Aufnahmeschlitz (44) einen, insbesondere quer zur Längserstreckung des Schiebteils (12) verlaufenden, vorzugsweise stab- oder stangenförmigen, Mitnehmerabschnitt (43) des Schiebteils (12) hintergreift, derart, dass das Schiebeteil (12) bei dessen Längsverschieben das Maulteil (39) um seine Schwenkachse (47) verschwenkt.

11. Chirurgisches Instrument nach Anspruch 10,
**dadurch gekennzeichnet,**
**dass** der Aufnahmeschlitz (44) derart konturiert ist, dass das Schiebeteil (12) bei entriegelten Verriegelungsmitteln (24) durch manuelle Betätigung des Maulteils (39) in Richtung einer Aufnahmeschlitzöffnung verstellt wird.

12. Chirurgisches Instrument nach einem der vorhergehenden Ansprüche,
**dadurch gekennzeichnet,**
**dass** der Mitnehmerabschnitt (43) zwischen zwei Seitenabschnitten (45, 46) des Schiebeschaftes aufgenommen ist oder ein freies Ende aufweist.

13. Chirurgisches Instrument nach einem der vorhergehenden Ansprüche,
**dadurch gekennzeichnet,**
**dass** die Führung (16) als, vorzugsweise im Schaft (2) vorgesehene und oben offene, T-Nut (42) ausgebildet ist.

14. Chirurgisches Instrument nach Anspruch 13,
**dadurch gekennzeichnet,**
**dass** ein distaler Abschnitt (30) des Schiebeteils (12) in der Arbeitsstellung, insbesondere in Richtung der Längserstreckung der Drehachse (21), von dem Schaft (2) weg, vorgespannt ist.

## Claims

1. Surgical instrument, in particular laminectomy rongeur or conchotome, comprising a shaft (2) and a sliding part (12), longitudinally displaceable relative to the shaft (2), to which, for longitudinal displacement a handle (6) pivotable around a pivot axis (5) is associated, wherein the sliding part (12) can be transferred from a working position, in which the sliding part (12) on the shaft (2) is longitudinally displaceably moved in a guide (16), into a cleaning position, in which the sliding part (12) is released from the guide (16) and can be pivoted relative to the shaft (2) around a rotation axis (21) oriented perpendicularly to the longitudinal extension of the shaft (2), said rotation axis (21) being aligned perpendicularly to the pivot axis (5) of the pivotable handle (6), **characterized in that** the sliding part (12) is captively locked on the shaft (2) in the working position, while being transferred from the working position into the cleaning position and in the cleaning position.

2. Surgical instrument according to claim 1,
**characterized in that** locking means (24) are provided, with which the sliding part (12) in the working position can be locked against inadvertent transfer into the cleaning position.

3. Surgical instrument according to claim 2,
**characterized in that** the locking means (24) are formed so as to adjust a check position for the pivotable handle (6).

4. Surgical instrument according to any one of claims 2 or 3,
**characterized in that** the locking means (24) have a locking pin (25) which can be adjusted preferably perpendicularly to the rotation axis (21), comprising a first diameter section (27) defining the check position for the pivotable handle (6) in the working position and a second diameter section (28) defining the check position for the pivotable handle (6) in the cleaning position.

5. Surgical instrument according to claim 4,
**characterized in that** the diameter of the first diameter section (27) is greater than the diameter of the second diameter section (28).

6. Surgical instrument according to any one of claims 4 or 5,
**characterized in that** the pivotable handle (6) is loaded by spring action against the locking pin (25).

7. Surgical instrument according to any one of claims 4 to 6,
**characterized in that** the locking pin (25) against the spring action of a spring (26) can be transferred from its locked position, in which the locking pin (25) locks the sliding part (12) in its working position, into a release position, in which the sliding part (12) can be pivoted around the rotation axis (21), and in which the check position for the pivotable handle (6) is adjusted relative to the check position for the pivotable handle (6) in the locked position.

8. Surgical instrument according to any one of the above claims,
**characterized in that** shaft (2) and sliding part (12) cooperate in the region of their distal working ends (3, 13) and/or **in that** the sliding part (12) is arranged so as to operate an active component, formed particularly as jaw part (39), preferably as jaw (40).

9. Surgical instrument according to claim 8,
**characterized in that** the sliding part (12) engages, preferably detachably, with the jaw part (39) at a distance from a pivot axis (47) of the jaw part (39).

10. Surgical instrument according to any one of claims 8 or 9,
**characterized in that** the jaw part (39) comprising a holding slot (44), in particular contoured in a diagonal or curved way, engages behind a preferably bar- or rod-shaped driver section (43), running especially transversely to the longitudinal extension of the sliding part (12), of the sliding part (12), in such a manner that the sliding part (12) when it is longitudinally displaced pivots the jaw part (39) around its pivot axis (47).

11. Surgical instrument according to claim 10,
**characterized in that** the holding slot (44) is contoured in such a manner that the sliding part (12), when the locking means (24) are unlocked, is adjusted by manual operation of the jaw part (39) towards a holding slot opening.

12. Surgical instrument according to any one of the above claims,
**characterized in that** the driver section (43) is held between two side sections (45, 46) of the sliding shaft or has a free end.

13. Surgical instrument according to any one of the above claims,
**characterized in that** the guide (16) is formed as a T-groove (42), preferably provided in the shaft (2) and open to the top.

14. Surgical instrument according to claim 13,
**characterized in that** a distal section (30) of the sliding part (12) in the working position, particularly towards the longitudinal extension of the rotation axis (21), is pre-tensioned away from the shaft (2).

## Revendications

1. Instrument chirurgical, notamment rongeur à laminectomie ou conchotome, comprenant une tige (2) et une partie coulissante (12) déplaçable longitudinalement par rapport à la tige (2), à laquelle est associée, pour le déplacement longitudinal, une partie de préhension (6) pouvant pivoter autour d'un axe de pivotement (5), la partie coulissante (12) pouvant être transférée d'une position de travail dans laquelle la partie coulissante (12) est guidée de manière déplaçable longitudinalement sur la tige (2) dans un guide (16), dans une position de nettoyage dans laquelle la partie coulissante (12) est libérée du guide (16) et peut pivoter par rapport à la tige (2) autour d'un axe de rotation (21) orienté perpendiculairement à l'étendue longitudinale de la tige (2), l'axe de rotation (21) étant orienté perpendiculairement à l'axe de pivotement (5) de la partie de préhension pivotante (6),
**caractérisé en ce que** la partie coulissante (12) est fixée de manière imperdable sur la tige (2) dans la position de travail, pendant le transfert de la position de travail dans la position de nettoyage et dans la position de nettoyage.

2. Instrument chirurgical selon la revendication 1,
**caractérisé en ce que** des moyens de verrouillage (24) sont prévus, avec lesquels la partie coulissante (12) peut être fixée dans la position de travail pour empêcher son transfert accidentel dans la position de nettoyage.

3. Instrument chirurgical selon la revendication 2,
**caractérisé en ce que** les moyens de verrouillage (24) sont réalisés de manière à régler une position de butée pour la partie de préhension pivotante (6).

4. Instrument chirurgical selon l'une quelconque des revendications 2 ou 3,
**caractérisé en ce que**
les moyens de verrouillage (24) présentent un boulon de verrouillage (25) réglable, de préférence perpendiculairement à l'axe de rotation (21), avec une première portion de diamètre (27) définissant la position de butée pour la partie de préhension pivotante (6) dans la position de travail et une deuxième portion de diamètre (28) définissant la position de butée pour la partie de préhension pivotante (6) dans la position de nettoyage.

5. Instrument chirurgical selon la revendication 4,
**caractérisé en ce que** le diamètre de la première portion de diamètre (27) est supérieur au diamètre de la deuxième portion de diamètre (28).

6. Instrument chirurgical selon l'une quelconque des revendications 4 ou 5,
**caractérisé en ce que** la partie de préhension pivotante (6) est sollicitée par la force d'un ressort contre le boulon de verrouillage (25).

7. Instrument chirurgical selon l'une quelconque des revendications 4 à 6,
**caractérisé en ce que** le boulon de verrouillage (25) peut être transféré, à l'encontre de la force de ressort d'un ressort (26), depuis sa position de fixation dans laquelle le boulon de verrouillage (25) fixe la partie coulissante (12) dans sa position de travail, dans une position de libération dans laquelle la partie coulissante (12) peut tourner autour de l'axe de rotation (21) et dans laquelle la position de butée pour la partie de préhension pivotante (6) est réglée par rapport à la position de butée pour la partie de préhension pivotante (6) dans la position de fixation.

8. Instrument chirurgical selon l'une quelconque des revendications précédentes, **caractérisé en ce que**
la tige (2) et la partie coulissante (12) coopèrent dans la région de leurs extrémités de travail distales (3, 13), et/ou **en ce que** la partie coulissante (12) est disposée de manière à actionner une partie d'organe fonctionnel réalisée notamment sous forme de partie de mâchoire (39) d'un organe fonctionnel réalisé de préférence sous forme de mâchoire (40).

9. Instrument chirurgical selon la revendication 8,
**caractérisé en ce que** la partie coulissante (12) est en prise, de préférence de manière détachable, avec la partie de mâchoire (39), à distance d'un axe de pivotement (47) de la partie de mâchoire (39).

10. Instrument chirurgical selon l'une quelconque des revendications 8 ou 9,
**caractérisé en ce que** la partie de mâchoire (39) vient en prise par l'arrière, par une fente de réception (44) ayant notamment un contour oblique ou courbe, avec une portion d'entraînement (43) de la partie coulissante (12) s'étendant notamment transversalement à l'étendue longitudinale de la partie coulissante (12), de préférence en forme de barre ou de tige, de telle sorte que la partie coulissante (12), lors de son déplacement longitudinal, fasse pivoter la partie de mâchoire (39) autour de son axe de pivotement (47).

11. Instrument chirurgical selon la revendication 10,
**caractérisé en ce que** la fente de réception (44) a un contour tel que la partie coulissante (12) soit réglée, lorsque les moyens de verrouillage (24) sont déverrouillés, par l'actionnement manuel de la partie de mâchoire (39) dans la direction d'une ouverture de la fente de réception.

12. Instrument chirurgical selon l'une quelconque des revendications précédentes, **caractérisé en ce que**
la portion d'entraînement (43) est reçue entre deux portions latérales (45, 46) de la tige coulissante ou présente une extrémité libre.

13. Instrument chirurgical selon l'une quelconque des revendications précédentes, **caractérisé en ce que**
le guide (16) est réalisé sous forme de rainure en T (42) prévue de préférence dans la tige (2) et ouverte vers le haut.

14. Instrument chirurgical selon la revendication 13,
**caractérisé en ce qu'**une portion distale (30) de la partie coulissante (12) est précontrainte dans la position de travail, notamment dans la direction de l'étendue longitudinale de l'axe de rotation (21), à l'écart de la tige (2).
